# EUROPEAN PATENT APPLICATION

(11) **EP 3 281 582 A1**
(43) Date of publication of application: **14.02.2018**
(21) Application number: 16776722.7
(22) Date of filing: 05.04.2016
(51) Int. Cl.: A61B 5/0484, A61B 5/0476

(54) **METHOD FOR ESTIMATING PERCEPTUAL SEMANTIC CONTENT BY ANALYSIS OF BRAIN ACTIVITY**

(30) Priority: 06.04.2015 JP 2015077694
(71) Applicant: National Institute of Information and Communications Technology, Koganei-shi Tokyo 184-8795 (JP)
(72) Inventor: NISHIMOTO, Shinji, Koganei-shi, Tokyo 184-8795 (JP); KASHIOKA, Hideki, Koganei-shi, Tokyo 184-8795 (JP)
(74) Representative: Strehl Schübel-Hopf & Partner
(86) International application number: PCT/JP2016/061645
(87) International publication number: WO 2016/163556

(57) **Abstract**

Provided is an estimation method for measuring and analyzing brain activity to estimate a perceptual semantic content thereof. This method comprises: (A) inputting, to a data processing means, brain activity induced in a subject by a training stimulation and detected as an output of a brain activity detection means and an annotation of a perceptual content; (B) associating a sematic space representation of the training stimulation and the output of the brain activity detection means in a stored semantic space and storing the association in a training result information storage means; (C) inputting, to the data processing means, an output when the brain activity detection means detects brain activity induced by a novel stimulation, and obtaining a probability distribution in the semantic space which represents perceptual semantic contents for the output of the novel stimulation-induced brain activity by the brain activity detection means on the basis of the association; and (D) estimating a highly probable perceptual semantic content on the basis of the probability distribution. The association process may be performed for each subject in the probability estimation process, the likelihood calculated on the basis of the coordinate of a given word in the semantic space and the probability distribution is used as an indicator.

## Description

### Technical Field

The present invention relates to a method for estimating a perceptual semantic content by analysis of brain activity to estimate a perceptual semantic content perceived by a subject by measurement of brain activity of the subject in a natural perception state during viewing a movie clip or the like and by analysis of the measured information.

### Background Art

Technologies for estimating a perceptual content and predicting an action by analysis of brain activity of a subject (brain information decoding technology) have been developed. These technologies are expected as an elemental technology of a brain-machine interface and as a means for prior assessment of a video or other products, prediction of purchasing, and the like.

The current semantic perception estimation technology based on brain activity is restricted for estimating a predetermined perceptual semantic content for restricted perception targets such as a simple line drawing and a still image including a single perceptual semantic content or a few perceptual semantic contents.

The procedure for decoding a perceptual semantic content on the basis of brain activity by using the conventional technology is as follows. First, model training (calibration) for interpreting a person's brain activity is performed. At this stage, a set of stimulations including images and the like is presented to a subject, and brain activity induced by these stimulations is recorded. On the basis of stimulation-brain activity pairs (training data samples), associations between a perceptual content and brain activity are obtained. Subsequently, novel brain activity that is a target for estimating a perceptual semantic content is recorded, and it is determined which of the brain activities obtained as the training data samples is similar to the novel brain activity, thereby estimating a perceptual semantic content.

PTL 1 discloses interpreting and reconstructing a subjective perceptual or cognitive experience. In this disclosure, a first set of brain activity data produced in response to a first perceptual stimulation is obtained from a target by using a brain imaging apparatus and is converted into a corresponding set of predetermined response values. A second set of brain activity data produced in response to a second perceptual stimulation is obtained from a target by using a decoding distribution, and a probability as the second set of brain activity data corresponds to the predetermined response values is determined. The second set of brain activity stimulations is interpreted on the basis of the probability of correspondence between the second set of brain activity data and the predicted response values.

NPL 1 describes encoding and decoding by using fMRI (functional Magnetic Resonance Imaging). This literature illustrates that encoding and decoding operations can both be used to investigate some of the most common questions about how information is represented in the brain. However, focusing on encoding models offers two important advantages over decoding. First, an encoding model can in principle provide a complete functional description of a region of interest, while a decoding model can provide only a partial description. Second, while it is straightforward to acquire an optimal decoding model from an encoding model, it is much more difficult to acquire an encoding model from a decoding model. Thus, NPL 1 proposes a systematic modeling approach that begins by estimating an encoding model for voxel in an fMRI scan and ends by using the estimated encoding models to perform decoding.

In addition, it has already been reported that it is possible to take brain images acquired during viewing a scene and to reconstruct an approximation of the scene from those images. NPL 2 further illustrates that it is also possible to generate text about the mental content reflected in brain images. This begins with brain images collected as subjects read names of concrete items (e.g., "Apartment") while also seeing line drawings of the item names. A model of the mental semantic representation of concrete concepts is built from text data, and aspects of such representation of patterns of activation are mapped in the corresponding brain image. It is reported that from the mapping, a collection of semantically pertinent words (e.g., "door", "window" for "apartment") was able to be generated.

### Citation List

### Patent Literature

PTL 1: U.S. Patent Application Publication No. 2013-0184558

### Non Patent Literature

NPL 1: Thomas Naselaris, Kendrick N. Kay, Shinji Nishimoto, Jack L. Gallant, "Encoding and decoding in fMRI", NeuroImage 2011, 56(2):400-410
NPL 2: Francisco Pereira, Greg Detre, Matthew Botvinick "Generating text from functional brain images", Frontiers in Human Neuroscience 2012, 5:72

### Summary of Invention

### Technical Problem

The technology that is an object of the present invention enables estimating an arbitrary perceptual semantic content of a subject in a natural perception state such as viewing a movie clip. In this respect, the conventional technology has reached its limitation in at least one of the following points and has not been capable of achieving the object. (1) The target of the conventional technology is a simple line drawing or a still image, and the conventional technology is not applicable to a situation in which a large number of things, impressions, and the like dynamically occur, such as in a natural movie clip. (2) In the conventional technology, a perceptual semantic content that can be estimated is limited to what is included in the training data samples, and other arbitrary perceptual semantic contents cannot be estimated.

### Solution to Problem

The technology that is the object of the present invention includes estimating a perceptual semantic content perceived by a subject, by analysis of measured information as described above. In this case, estimating an arbitrary perceptual content is realized by associating brain activity with a perceptual content in an internal representation space (semantic space). Details will be described below.

A method for estimating a perceptual semantic content by analysis of brain activity according to the present invention is a method for estimating a perceptual semantic content perceived by a subject with analysis of brain activity of the subject and with a use of a brain activity analysis apparatus that includes: an information presenting means for presenting information serving as a stimulation for the subject; an brain activity detection means for detecting a brain activity signal of the subject caused by the stimulation; a data processing means that inputs an annotation related to a stimulation content and an output of the brain activity detection means; a semantic space information storage means from which data is readable by the data processing means; and a training result information storage means from and to which data is readable and writable by the data processing means.
(1) Training information is presented to the subject to give the subject a training stimulation, and an annotation of a perceptual content induced in the subject by the training stimulation and an output from the brain activity detection means that detects brain activity induced in the subject by the training stimulation are input to the data processing means.
   Here, the training information is an image, a movie clip, or the like, the information serves as a stimulation for the subject, and the stimulation induces a certain perceptual content in the subject. An annotation of the perceptual content is acquired and input to the data processing means. In addition, the output when the brain activity detection means detects brain activity as an electroencephalogram or fMRI signals is also input to the data processing means.
(2) A semantic space stored in the semantic space information storage means is applied, a semantic space representation of the training stimulation and the output of the brain activity detection means are associated in the semantic space, and a result of the association is stored in the training result information storage means.
   The semantic space is constructed by using a large-scale database such as a corpus, in which semantic relationships between the words appearing in the annotation are described.
   In addition, the association is performed on coordinate axes of the semantic space and herein refers to associations between a semantic space representation induced by a stimulation using the training information and brain activity caused by the stimulation.
(3) Novel information is presented to the subject to give the subject a novel stimulation, an output from the brain activity detection means that detects brain activity induced in the subject by the novel stimulation is input to the data processing means, and a probability distribution in the semantic space that represents perceptual semantic contents for the output of brain activity from the brain activity detection means, the brain activity having been caused by the novel information, is obtained on the basis of the association obtained in (2).
   The output of the brain activity detection means, such as an electroencephalogram or fMRI signals, for the brain activity induced by the novel stimulation is decomposed as, for example, a linear synthesis of the output of the brain activity detection means induced by the training stimulation or a signal or an ignition pattern extracted therefrom, and thereby a perceptual semantic content in response to the novel stimulation can be obtained as a linear synthesis of an annotation corresponding to the training information. On the basis of a coefficient of the linear synthesis and the association obtained in (2), a probability distribution in the semantic space that represents the perceptual semantic contents in response to the novel stimulation can be obtained.
(4) A highly probable perceptual semantic content is estimated on the basis of the probability distribution obtained in (3).

In the estimation, for example, by setting a threshold of the probability used for the estimation based on the probability distribution or by setting a threshold of the number of highly probable perceptual semantic contents, divergence of the estimation results can be suppressed.

If there are a plurality of subjects, the association between the semantic space representation of the stimulation and the brain activity by using the training data in (2) may be performed for each of the subjects for all or a part of the training data, a projection function for each of the subjects may be obtained, and in accordance with the projection function, association with a location in the semantic space may be uniformly differentiated for each of the subjects.

When the highly probable perceptual semantic content is estimated in (4), a coordinate in the semantic space for a given arbitrary word can be found, a likelihood between the coordinate and the probability distribution obtained in (3) can be calculated, and a value of the likelihood can be set as an indicator of the probability.

### Advantageous Effects of Invention

According to the present invention, it becomes possible to estimate an arbitrary perceptual semantic content in a natural perception state of a movie clip or the like on the basis of brain activity.

### Brief Description of Drawings

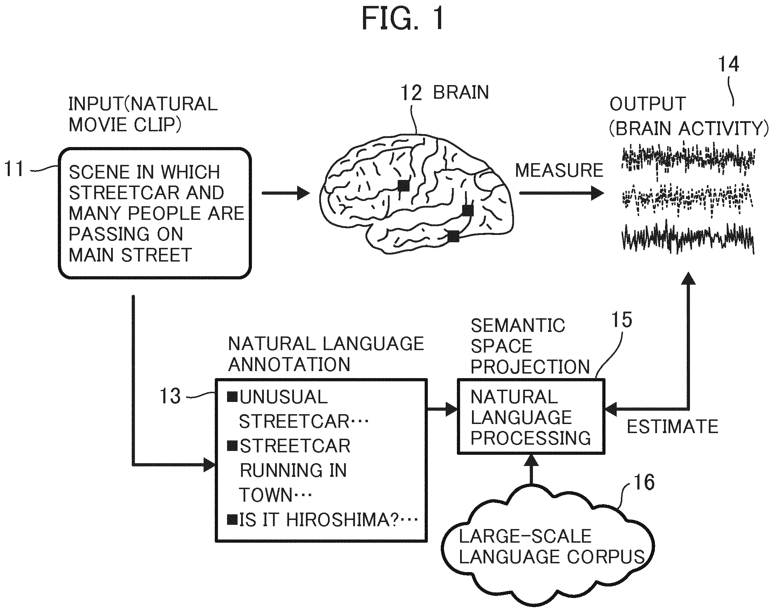

Fig. 1 is a conceptual view of estimation of a semantic space model and a perceptual semantic content of brain activity. Fig. 1 illustrates that the correspondence relationship between brain activity and a semantic space derived from a corpus is learnt as a quantitative model to estimate a perceptual semantic content on the basis of brain activity under arbitrary novel conditions.

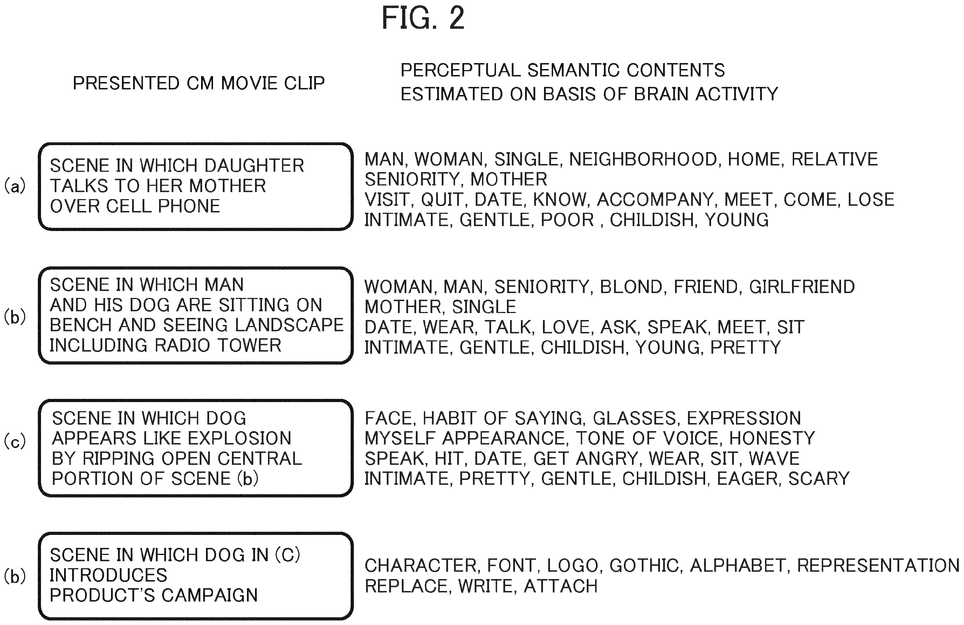

Fig. 2 illustrates an example of estimating perceptual semantic contents on the basis of brain activity during viewing a television commercial (CM) movie clip. (Left) illustrates CM clip examples presented to a subject, and (Right) illustrates perceptual semantic contents estimated on the basis of brain activity during viewing the corresponding clips. Each row beside the clips lists words according to parts of speech such as nouns, verbs, and adjectives that may highly possibly be perceived.

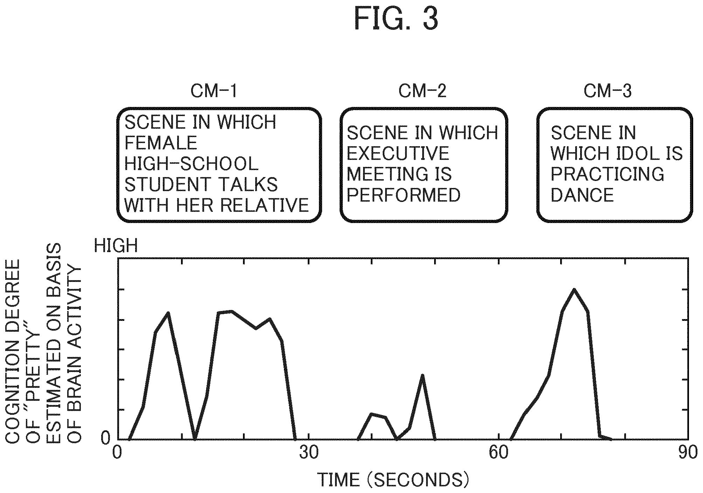

Fig. 3 illustrates a quantitative evaluation example based on brain activity in a time series of a specific impression. The degree of cognition of a specific impression ("pretty" in this case) is estimated on the basis of brain activity in brain activity during viewing three 30-second CMs.

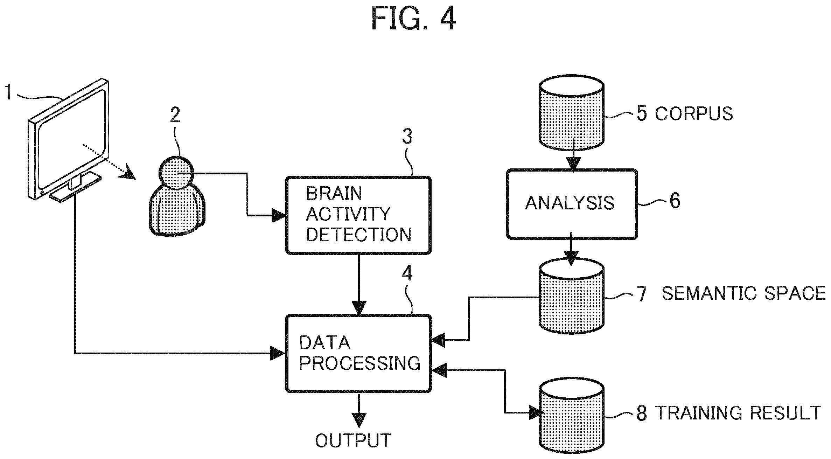

Fig. 4 illustrates an apparatus configuration example for applying the present invention.

### Description of Embodiments

Hereinafter, embodiments of the present invention will be described in detail with reference to the drawings.

### Embodiment 1

Fig. 4 illustrates an apparatus configuration example for applying the present invention. A display apparatus 1 presents a training stimulation (e.g., an image or a movie clip) to a subject 2, and brain activity signals of the subject 2 are detected by a brain activity detection unit 3 that can detect, for example, an EEG (electroencephalogram) or fMRI signals. As the brain activity signals, an ignition pattern of brain cells or a signal of activity change in one or more specific regions is detected. The detected brain activity signals are processed by a data processing apparatus 4. In addition, a natural language annotation from the subject 2 is input to the data processing apparatus 4. A semantic space used for data processing is obtained by an analysis apparatus 6 analyzing corpus data from a storage 5 and is stored in a storage 7.

As for the training stimulation, natural language annotation data from the subject 2 or a third party is analyzed by the data processing apparatus 4 serving as a vector in the semantic space, and the analysis result is stored in a storage 8 as a training result in addition to the brain activity signals of the subject 2.

If a novel stimulation is presented to the subject 2 through the display apparatus 1, the brain activity detection unit 3 detects brain activity signals, and the data processing apparatus 4 analyzes the signals on the basis of the semantic space from the storage 7 and the training result from the storage 8, and the analysis result is output from the data processing apparatus 4.

Here, the storage 5, the storage 7, and the storage 8 may be obtained by dividing one storage region, and the data processing apparatus 4 and the analysis apparatus 6 may be used by switching one computer.

In a method for estimating a perceptual semantic content by analysis of brain activity according to the present invention, brain information decoding is performed through a semantic space derived from a corpus. Thus, an arbitrary perceptual semantic content is interpreted on the basis of brain activity. A more specific procedure is as follows, as will be described with reference to Fig. 1. Fig. 1 is a conceptual view of estimation of a semantic space model and a perceptual semantic content of brain activity. Fig. 1 illustrates an outline of a procedure in which the correspondence relationship between brain activity and a semantic space derived from a corpus is learnt as a quantitative model to estimate a perceptual semantic content on the basis of brain activity under arbitrary novel conditions.
(a) Annotations 13 of perceptual contents induced in a subject by a training stimulation 11 (e.g., an image or a movie clip) are acquired.
   More specifically, a certain still image or movie clip (training data) is presented to a subject 12 as a training stimulation, and a list of annotations that the subject has in response to the presentation is created.
(b) A semantic space for describing semantic relationships of the words appearing in the annotations is constructed by using a large-scale database such as a corpus 16. It is well known that a natural language processing technology such as Latent Semantic Analysis, word2vec or the like is used as a method for constructing a semantic space from a corpus.
   As the corpus, newspaper and magazine articles, encyclopedias, tales, and the like can be used. Here, as is well known, the semantic space derived from a corpus is a space for projecting elements such as words into a fixed-length vector space on the basis of statistical characteristics inherent in a corpus. As a matter of course, if a semantic space has already been obtained, the semantic space can be used.
   In addition, Latent Semantic Analysis is a well-known method and a principal component analysis method in which singular value decomposition is performed on a co-occurrence matrix indicating the words included in a sentence object that is an analysis target, and dimension reduction is then performed to acquire the main semantic structure of a target text.
   In addition, Word2Vec is a quantification method for representing words as vectors. In Word2Vec, a word appearance prediction model of a sentence is optimized, and thereby fixed-length vector space representations of the words are learnt.
(c) In the semantic space obtained in the above (b), the stimulation 11 is subjected to semantic space projection 15 by using the training data, and the representations in the semantic space are associated with a brain activity output 14.
   The training data (e.g., an image or a movie clip) is presented to the subject, and brain activity signals, for example an EEG (electroencephalogram) or fMRI signals, generated in response are detected. The detected brain activity signals are associated with the location in the semantic space. In this association, the representations in the above semantic space are associated with the signal waveform of an EEG (electroencephalogram) or fMRI.
   It is desirable that this association be performed for each subject. However, the association at this time does not have to be performed for all of the pieces of the training data. The association for some pieces of the training data may be performed to obtain a projection function in the semantic space for each subject, and in accordance with the projection function, the association with the location in the semantic space may be uniformly differentiated.
(d) For novel brain activity, on the basis of the association obtained in the above (c), a probability distribution in the semantic space representing a perceptual semantic content is obtained.
   Novel data (e.g., an image or a movie clip) is presented to the subject, and brain activity signals are detected by using a brain activity signal acquiring means that has been used for the above training data. The detected brain activity signals are compared with the brain activity signals obtained for the training data, and it is determined which of the brain activity signals for the training data is similar to the brain activity signals for the novel data. Alternatively, it is determined what kind of mixture of the brain activity signals for the training data is similar to the brain activity signals for the novel data. This comparison can be performed by using, as an indicator, for example, a peak value of cross-correlation between the brain activity signals for the novel data and the brain activity signals for the training data. With this determination, a probability distribution corresponding to the brain activity signals detected in response to the presentation of the novel data in a semantic space can be obtained.
(e) On the basis of the probability distribution obtained in the above (d), a highly probable perceptual semantic content is estimated.
   In the above (a), the annotations of perceptual contents corresponding to the training data are obtained, and in the above (b), each word is represented as a vector in a semantic space. Accordingly, in the semantic space, on the basis of the probability distribution corresponding to the brain activity signals, the annotations of perceptual contents corresponding to the brain activity signals can be obtained with probability weighting. By using the probability weighting, a highly probable annotation is estimated.
   Here, since the perceptual contents induced in the subject by a stimulation are represented as annotations using the list in the above (a), the list desirably covers all or a selected predetermined part of the semantic space derived from a corpus.
   In the above manner, the present invention provides a technology for estimating an arbitrary perceptual semantic content perceived by a subject, on the basis of brain activity in a state of perception of relatively dynamic and complex audio-visual content such as a television commercial (CM). With the present invention, on the basis of brain activity in a natural perception state of a movie clip or the like, an arbitrary perceptual semantic content can be estimated. For example, quantitative evaluation based on brain activity is enabled to determine whether a movie clip production such as the above television commercial exhibits expression effects as aimed.

### Embodiment 2

A topic model of LDA (Latent Dirichlet Allocation) can be applied to handle the annotations in the above embodiment 1. Thus, it becomes easy to estimate a perceptual semantic content on the basis of the estimated brain activity and to represent the perceptual semantic content as a sentence. An example procedure for this will be described below.
(A) Annotations 13 of perceptual contents induced in a subject by a training stimulation 11 (e.g., an image or a movie clip) are acquired.
   More specifically, a certain still image or movie clip (training data) is presented to a subject 12 as a training stimulation, and a list of annotations that the subject has in response to the presentation is created.
(B) A topic model for describing semantic relationships of the words appearing in the annotations is constructed by using a large-scale database such as a corpus 16. The topic model can be prepared by a well-known method such as LDA. As is well known, the topic model is a statistical model, and an appearance probability of each word can be obtained.
(C) In the topic model obtained in the above (B), the training data is replaced by labels of a topic to which morphemes of the training data belongs, and the labels are associated with a brain activity output 14.
   That is, the training data (e.g., an image or a movie clip) is presented to the subject, and brain activity signals, such as an EEG (electroencephalogram) or fMRI signals, generated in response are detected. The detected brain activity signals are associated with the labels of the topic to which the morphemes of the training data belong. In this association, brain activity signals for one piece of training data may be associated with, for example, a linear combination of labels, or, in contrast, one label may be associated with a linear combination of brain activity signals.
   It is desirable that this association be performed for each subject. However, the association at this time does not have to be performed for all of the pieces of the training data. The association for some pieces of the training data may be performed, and some association processes can be omitted.
(D) For novel brain activity, on the basis of the association obtained in the above (C), a probability distribution in the annotation typified by the label of the topic model representing a perceptual semantic content is obtained.
   Novel data (e.g., an image or a movie clip) is presented to the subject, and brain activity signals are detected by using a brain activity signal acquiring means that has been used for the above training data. The detected brain activity signals are compared with the brain activity signals obtained for the training data, and it is determined which of the brain activity signals for the training data is similar to the brain activity signals for the novel data. Alternatively, it is determined what kind of mixture of the brain activity signals for the training data is similar to the brain activity signals for the novel data. This comparison can be performed by using, as an indicator, for example, a peak value of cross-correlation between the brain activity signals for the novel data and the brain activity signals for the training data. With this determination, a probability distribution of the annotations corresponding to the brain activity signals detected in response to the presentation of the novel data can be obtained.
(E) On the basis of the probability distribution obtained in the above (D), a highly probable perceptual semantic content is estimated.

In the case of this embodiment, since the probability distribution of the annotations has been obtained in the above (D), a sentence can be estimated by a method like LDA.

Here, since the perceptual contents induced in the subject by a stimulation in (A) is represented as annotations using the list in the above (a), the list desirably covers all or a selected predetermined part of the semantic space derived from a corpus.

The present invention provides a technology for estimating an arbitrary perceptual semantic content perceived by a subject on the basis of brain activity in a state of perception of relatively dynamic and complex audio-visual content for example a television commercial (CM). With the present invention, on the basis of brain activity in a natural perception state of a movie clip or the like, an arbitrary perceptual semantic content can be estimated. For example, quantitative evaluation based on brain activity is enabled to determine whether a movie clip production such as the above television commercial exhibits expression effects as aimed.

### Embodiment 3

The example illustrated in Fig. 2 is an estimation example of perceptual semantic contents on the basis of brain activity during viewing a CM movie clip. Specifically, an object is, for example, to reasonably reply to a question as to how audience's perception of "intimacy" is induced. This illustrates perceptual semantic contents estimated on the basis of brain activity through the procedure of the above (a) to (e) with respect to the presented CM movie clip in FIG. 2. The left column illustrates CM clip examples presented to a subject, and the right column illustrates perceptual semantic contents estimated on the basis of brain activity during viewing the corresponding clips. Each row beside the clips lists words according to parts of speech such as nouns, verbs, and adjectives in descending order of probability that the subject may perceive.
Fig. 2(a): A scene in which a daughter talks to her mother over a cell phone
   (noun) man, woman, single, neighborhood, home, relative, seniority, mother
   (verb) visit, quit, date, know, accompany, meet, come, lose (adjective) intimate, gentle, poor, childish, young
Fig. 2(b): A scene in which man and his dog are sitting on a bench and seeing the landscape including a radio tower
   (noun) woman, man, seniority, blond, friend, girlfriend, mother, single
   (verb) date, wear, talk, love, ask, speak, meet, sit (adjective) intimate, gentle, childish, young, pretty
Fig. 2(c): A scene in which the dog appears like an explosion by ripping open a central portion of the scene (b)
   (noun) face, habit of saying, glasses, expression, myself, appearance, tone of voice, honesty
   (verb) speak, hit, date, get, angry, wear, wear, sit, wave (adjective) intimate, pretty, gentle, childish, eager, scary
Fig. 2(d): A scene in which the dog in (c) introduces a product's campaign
   (noun) character, font, logo, gothic, alphabet, representation
   (verb) replace, write, attach

It becomes possible to objectively determine whether these perceptual semantic contents representing the audience's brain activity accord with the creators of the CM.

In addition, sentences can be estimated through the procedure in the above (A) to (E).

### Embodiment 4

The example in Fig. 3 illustrates a quantitative evaluation example based on brain activity in a time series of a specific impression. An object of this is, for example, to provide a quantitative indicator for which of two images A and B gives a stronger specific impression to audience. The degree of cognition of a specific impression ("pretty" in this case) is estimated by determination as to whether the specific impression is a highly probable annotation on the basis of a time series of brain activity in brain activity during viewing three 30-second CMs. It is found that a relatively strong response is obtained by CM-1 among CM-1: a scene in which a female high-school student talks with her relative, CM-2: a scene in which an executive meeting is performed, and CM-3: a scene in which an idol is practicing dance.

### Industrial Applicability

The present invention can be widely used as a base of prior assessment of audio-visual materials (e.g., video, music, and teaching materials) and a brain-machine interface through reading of perceptions and intentions of actions.

### Reference Signs List

1 display apparatus
2 subject
3 brain activity detection unit
4 data processing apparatus
5 storage
6 corpus data analysis apparatus
7, 8 storage
11 stimulation
12 subject
13 annotation
14 brain activity output
15 semantic space projection
16 corpus

## Claims

1. A method for estimating a perceptual semantic content perceived by a subject with analysis of brain activity of the subject and with a use of a brain activity analysis apparatus that includes:
an information presenting means for presenting information serving as a stimulation for the subject,
an brain activity detection means for detecting a brain activity signal of the subject caused by the stimulation;
a data processing means that inputs an annotation related to a stimulation content and an output of the brain activity detection means;
a semantic space information storage means from which data is readable by the data processing means, and
a training result information storage means from and to which data is readable and writable by the data processing means, the method comprising the steps of:
(1) presenting training information to the subject to give the subject a training stimulation and inputting to the data processing means an annotation of a perceptual content induced in the subject by the training stimulation and an output from the brain activity detection means that detects brain activity induced in the subject by the training stimulation;
(2) applying a semantic space stored in the semantic space information storage means, associating a semantic space representation of the training stimulation and the output of the brain activity detection means in the semantic space, and storing a result of the association in the training result information storage means;
(3) presenting novel information to the subject to give the subject a novel stimulation, inputting to the data processing means an output from the brain activity detection means that detects brain activity induced in the subject by the novel stimulation, and obtaining a probability distribution in the semantic space that represents perceptual semantic contents for the output of brain activity from the brain activity detection means, the brain activity having been caused by the novel information, on the basis of the association obtained in (2); and
(4) estimating a highly probable perceptual semantic content on the basis of the probability distribution obtained in (3).

2. The method for estimating a perceptual semantic content by analysis of brain activity according to Claim 1, wherein the association between the semantic space representation of the stimulation and the brain activity by using the training information in (2) for subjects is performed for each of the subjects using all or a part of the training information, a projection function in the semantic space for each of the subjects is obtained, and in accordance with the projection function, association with a location in the semantic space is transformed for each of the subjects.

3. The method for estimating a perceptual semantic content by analysis of brain activity according to Claim 1 or 2, wherein, when the highly probable perceptual semantic content is estimated in (4), a coordinate in the semantic space for a given arbitrary word is found, an inner product of the coordinate and the probability distribution obtained in (3) is calculated, and a value of the inner product is set as an indicator of the probability.
